# EUROPEAN PATENT APPLICATION

(11) **EP 3 838 413 A1**
(43) Date of publication of application: **23.06.2021**
(21) Application number: 20208745.8
(22) Date of filing: 19.11.2020
(51) Int. Cl.: B01L 3/02, B01L 3/00, A61M 1/36

(54) **SYRINGE INTEGRATED PLATELET EXTRACTION DEVICE**

(30) Priority: 18.12.2019 KR 20190169718
(71) Applicant: Wellfort Co., Ltd., Seoul 06097 (KR)
(72) Inventor: CHO, Hyung Jin, 06077 Seoul (KR); KIM, Keun Duk, 31971 Seosan-si, Chungcheongnam-do (KR); KIM, Teh Sob, 31971 Seosan-si, Chungcheongnam-do (KR)
(74) Representative: BCKIP

(57) **Abstract**

Disclosed herein is a syringe-integrated platelet extraction device. The syringe-integrated platelet extraction device includes: a housing having a longitudinal inner space for receiving collected blood, open at a top thereof, and formed at a bottom thereof with an orifice to which a syringe needle is coupled; a cap coupled to the orifice of the housing; a collector press-fitted into the housing from above the housing; a connector detachably coupled to an upper portion of the collector; and a plunger detachably coupled to the connector.

## Description

### FIELD

The present invention relates to a syringe-integrated platelet extraction device. More particularly, the present invention relates to a syringe-integrated platelet extraction device which is cheap to manufacture through integration of a blood collection instrument and a platelet extraction instrument, can simplify a platelet extraction procedure, can minimize contamination of blood due to exposure to the atmosphere, and can extract high-purity platelets.

### BACKGROUND

Blood carries oxygen from the lungs to tissue cells, carries carbon dioxide from tissue cells to the lungs to release the carbon dioxide into the atmosphere, and carries nutrients absorbed in the digestive tract to organs or tissue cells.

In addition, blood carries breakdown products of tissue, unnecessary for the body, to the kidneys to discharge the breakdown products from the body and carries hormones secreted from the endocrine glands to organs and tissue. Further, blood serves to maintain a constant body temperature by evenly distributing body heat and performs various other functions such as destroying and detoxifying bacteria and foreign substances that have invaded the body.

Although blood is used as a major indicator to detect various diseases or to monitor physical conditions, platelets in the blood, which are rich in growth factors, are used for therapeutic purposes.

Blood is composed of red blood cells, white blood cells, platelets, and the like. Thereamong, the platelets are mostly present in plasma. The plasma is divided into platelet-rich plasma (PRP) and platelet-poor plasma (PPP). Thereamong, PRP is widely used not only for improvement of wrinkles, scars, and pigment disorders of the skin, but also for therapeutic purposes, such as helping to generate cells by stimulating stem cells through transplantation into a pain area, especially, the knee joint, ligaments, muscles, and the like.

Since PRP is present in a trace amount (about 1%) in collected blood and is difficult to separate from red blood cells due to high viscosity thereof, a technology for extracting high-purity PRP free from red blood cells is actively studied.

Conventionally, a PRP separation vessel has usually been used as a test tube. However, recently, a centrifugation method is generally used in order to achieve quick separation while allowing each separated phase to form a complete layer and a platelet extraction device adapted to extract PRP from centrifuged blood is generally used. The most common type of such a platelet extraction device employs a method in which blood collected from the body using a blood collection syringe is introduced into the platelet extraction device, followed by centrifugation to separate the blood into three layers, and then a needle of a syringe is inserted into an upper portion of the extraction device to extract only a PRP layer.

Despite having advantages of a relatively simple PRP layer separation process, this method has a problem in that there is a high risk of contamination of blood due to exposure to the atmosphere since the blood collection syringe and the platelet extraction device are used individually and in that it is very difficult to separate only the PRP layer using the syringe needle.

Recently, there has been developed a platelet extraction instrument that uses a centrifugal housing as a main body of a blood collection syringe to eliminate the need to separately introduce collected blood into the centrifugal housing, as in the related art, thereby preventing contamination of blood.

However, such a platelet extraction instrument still has a problem in that extraction of centrifuged plasma and platelets requires a process of screwing a separate connector onto the housing and screwing a corresponding extraction syringe onto the connector after centrifugation, causing reduction in purity of platelets due to partial mixing between plasma, platelets, and red blood cells during plasma or platelet extraction and contamination of blood due to exposure to the atmosphere during connection of the connector to the housing.

In addition, this platelet extraction instrument has problems of difficulty in precisely extracting only platelets and occurrence of interlayer mixing due to generation of a vortex during the extraction process, like typical platelet extraction instruments.

### SUMMARY

Embodiments of the present invention have been conceived to solve such a problem in the art and it is an aspect of the present invention to provide a syringe-integrated platelet extraction device which is manufactured in the form of a blood collection syringe such that blood collection, centrifugation, and platelet extraction can be performed using a single instrument, has a simple structure, is cheap to manufacture, and can prevent not only contamination of blood due to exposure to the atmosphere, but also occurrence of a vortex during platelet extraction, thereby allowing extraction of high-purity platelets.

In accordance with one aspect of the present invention, there is provided a syringe-integrated platelet extraction device including: a housing having a longitudinal inner space for receiving collected blood, open at a top thereof, and formed at a bottom thereof with an orifice to which a syringe needle is coupled; a cap coupled to the orifice of the housing; a collector press-fitted into the housing from above the housing; a connector detachably coupled to an upper portion of the collector; and a plunger detachably coupled to the connector, wherein the collector includes a body movably disposed inside the housing, a coupling portion formed at an upper side of the body and allowing a lower end of the connector to be detachably coupled thereto, a cup portion inwardly recessed from a lower surface of the body and receiving platelets therein, a support portion extending from a lower end of the coupling portion and allowing a plasma extraction syringe or a platelet extraction syringe to be inserted thereinto and coupled thereto, and a communication portion having a smaller diameter than the support portion and allowing the cup portion to communicate with the support portion.

The cup portion may have a volume corresponding to an amount of platelets to be extracted.

The cup portion may include: a first curved portion formed at a lower inner periphery of the body to be curved in an inward direction of the body; an inclined portion upwardly extending from the first curved portion and inclined at a predetermined angle toward the support portion; and a second curved portion extending from an upper end of the inclined portion and curved toward the support portion.

The support portion may include: a first coupling portion downwardly extending from the coupling portion, tapered toward a lower end thereof, and supporting an outer periphery of a tip of the plasma extraction syringe or the platelet extraction syringe; and a second coupling portion downwardly extending from a lower end of the first coupling portion, having a smaller diameter than the first coupling portion, and allowing an orifice of the plasma extraction syringe or the platelet extraction syringe to be inserted thereinto and coupled thereto.

The collector may further include a soft gasket coupled to an outer surface of the body.

The gasket may have multiple annular protrusions formed on an outer surface thereof.

The housing may further include an annular ledge inwardly protruding from an upper inner surface thereof.

The syringe-integrated platelet extraction device may further include: a holder retaining the housing by allowing one side of an outer surface of the housing to be supported on one side of an upper surface thereof.

The holder may include a movement prevention portion formed at one side of an inner surface thereof to support the one side of the outer surface of the housing.

The present invention provides a syringe-integrated platelet extraction device which is manufactured in the form of a blood collection syringe such that blood collection, centrifugation, and platelet extraction can be performed using a single instrument, has a simple structure, is cheap to manufacture, and can prevent not only contamination of blood due to exposure to the atmosphere, but also occurrence of a vortex during platelet extraction, thereby allowing extraction of high-purity platelets.

### DRAWINGS

FIG. 1 is a perspective view of a syringe-integrated platelet extraction device according to one embodiment of the present invention.
FIG. 2 is an exploded perspective view of the syringe-integrated platelet extraction device of FIG. 1.
FIG. 3 is a sectional view of the syringe-integrated platelet extraction device of FIG. 1.
FIG. 4 is a sectional view of the syringe-integrated platelet extraction device of FIG. 1, with a syringe needle coupled to the housing thereof.
FIG. 5 is a sectional view of the syringe-integrated platelet extraction device of FIG. 1, with blood collected in the housing thereof.
FIG. 6 is a sectional view of the syringe-integrated platelet extraction device of FIG. 1, showing centrifugation of blood collected in the housing thereof.
FIG. 7 is a sectional view of the syringe-integrated platelet extraction device of FIG. 1, showing extraction of plasma in the housing thereof.
FIG. 8 is a sectional view of the syringe-integrated platelet extraction device of FIG. 1, showing extraction of platelets in the housing thereof.

### DETAILED DESCRIPTION

Hereinafter, embodiments of the present invention will be described with reference to the accompanying drawings.

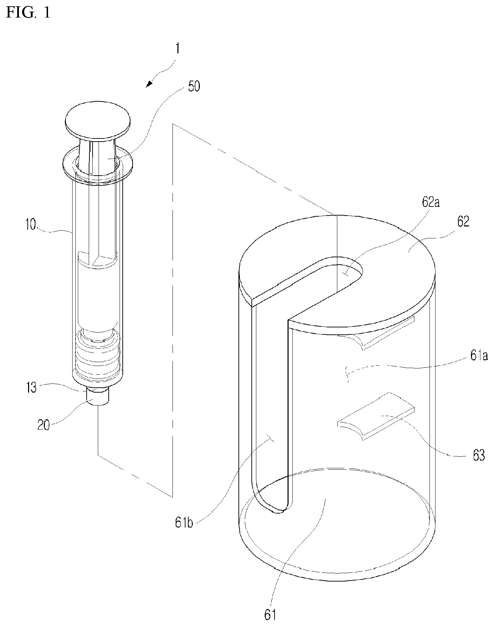

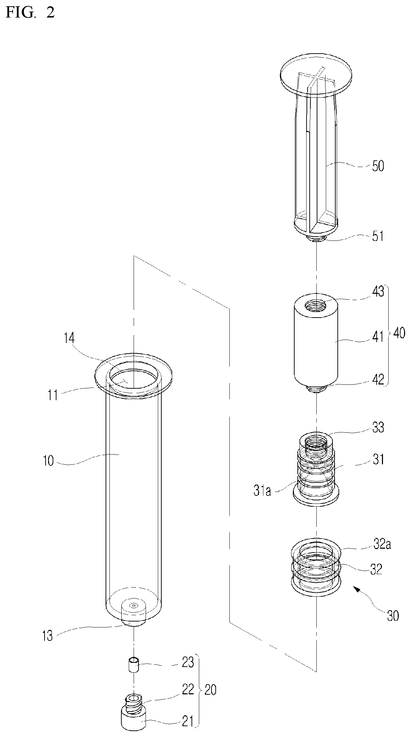

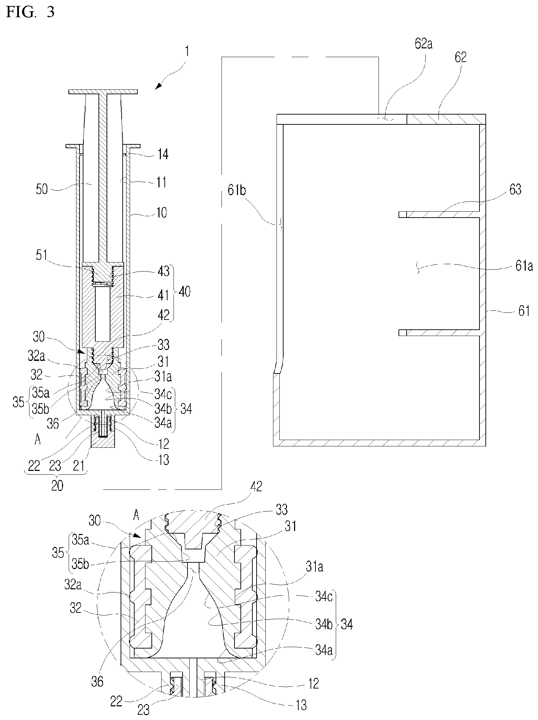

FIG. 1 is a perspective view of a syringe-integrated platelet extraction device according to one embodiment of the present invention, FIG. 2 is an exploded perspective view of the syringe-integrated platelet extraction device of FIG. 1, and FIG. 3 is a sectional view of the syringe-integrated platelet extraction device of FIG. 1.

Referring to FIG. 1 to FIG. 3, a syringe-integrated platelet extraction device according to one embodiment of the present invention includes a housing 10, a cap 20, a collector 30, a connector 40, a plunger 50, and a holder 60.

The housing 10 is in form of a cylinder having a predetermined length, has a longitudinal inner space 11 for receiving blood, and is open at a top thereof to expose the space 11.

In addition, the housing 10 has a hollow orifice 12 formed at a bottom thereof and coupled to a syringe needle or the cap 20 and a support wall 13 formed outside the orifice 12, open at a bottom thereof, and having a threaded inner surface to be coupled to the syringe needle or the cap 20.

Further, the housing 10 has an annular ledge 14 inwardly protruding from an upper inner surface thereof to prevent the collector 30 from being separated from the housing 10 and to identify or restrict a withdrawal height of the plunger 50 such that an appropriate amount of blood can be collected.

The housing 10 according to this embodiment may be any typical syringe housing 10. Depending on the amount of blood to be extracted, a syringe having a volume of 10 cc to 20 cc may generally be used.

The cap 20 includes a cylindrical main body 21, a coupling protrusion 22 protruding from an upper surface of the main body 21, having a threaded outer surface, and formed at upper end thereof with a vertical insertion hole 22a into which the orifice 12 of the housing 10 is inserted, and a packing member 23 disposed in the insertion hole 22a of the coupling protrusion 22 and sealing a gap between the orifice 12 and the coupling protrusion 22.

The coupling protrusion 22 of the cap 20 is screwed onto the support wall 13 of the housing 10 and the packing member 23 of the cap 20 seals the orifice 12 to prevent blood received in the housing 10 after blood collection from leaking outside.

The collector 30 includes a body 31 press-fitted into the housing 10, a gasket 32 coupled to an outer surface of the body 31, a coupling portion 33 formed at an upper side of the body 31, a cup portion 34 formed on a lower surface of the body 31, a support portion 35 formed inside the body 31, and a communication portion 36 through which the cup portion 34 communicates with the support portion 35.

The body 31 is in form of a cylinder having a predetermined length and has a support groove 31a formed on the outer surface thereof to support the gasket 32. The body 31 is press-fitted at one side thereof into the housing 10 and is coupled at the other side thereof to a plasma extraction syringe or a platelet extraction syringe to connect the housing 10 to the syringe.

The gasket 32 is coupled to the outer surface of the body 31 to be supported at one side thereof on the support groove 31a and held against the inner wall of the housing 10, thereby preventing blood from leaking through a gap between the body 31 and the inner wall of the housing 10 during extraction of plasma or platelets.

Preferably, the gasket 32 has at least three annular protrusions 32a formed on the outer surface thereof to be vertically separated from one another at regular intervals so as to provide improved sealing between the body 31 and the inner wall of the housing 10.

The coupling portion 33 is inwardly recessed from the upper surface of the body portion 31 and has a threaded inner surface to be screwed onto the connector 40 described below. The coupling portion 33 serves to allow the connector 40 to be detachably coupled to the body 31.

The cup portion 34 is inwardly recessed from the lower surface of the body 31 and has a volume corresponding to the amount of platelets to be extracted. In this embodiment, the cup portion 34 has a volume of 1 cc based on the assumption that 20 cc of blood is to be extracted.

The cup portion 34 makes it easy to identify a boundary between plasma and platelets during plasma extraction and the amount of extracted platelets while preventing occurrence of a vortex due to extraction pressure during plasma or platelet extraction, thus preventing mixing between plasma and platelets or between platelets and red blood cells.

More specifically, the cup portion 34 includes a first curved portion 34a formed on a lower inner surface of the body 31 to be curved in an inward direction of the body 31, an inclined portion 34b upwardly extending from the first curved portion 34a to be inclined at a predetermined angle toward the support portion 35, and a second curved portion 34c extending from an upper end of the inclined portion 34b to be curved toward the support portion 35.

That is, the cup portion 34 has a generally bell shape, whereby, when blood in the housing 10 is moved to the cup portion 34 having a relatively small diameter during plasma or platelet extraction, the blood can flow naturally into the cup portion 34 along the curved surface while occurrence of a vortex at an entrance of the cup portion 34 can be suppressed.

The support portion 35 includes a first coupling portion 35a extending from a lower end of the coupling portion 33 to be tapered toward a lower end thereof and a second coupling portion 35b extending from the lower end of the first coupling portion 35a and having a smaller diameter than the first coupling portion 35a.

Here, the first coupling portion 35a supports an outer periphery of a tip of the plasma extraction syringe or the platelet extraction syringe during plasma or platelet extraction, and the second coupling portion 35b securely receives and firmly supports an orifice of the plasma extraction syringe or the platelet extraction syringe, thereby minimizing movement of the plasma extraction syringe or the platelet extraction syringe during plasma or platelet extraction.

The communication hole 36 has a smaller diameter than the second coupling portion 35b and serves to connect the support portion 35 to the cup portion 34 to allow plasma or platelets in the housing 10 to be moved into the plasma extraction syringe or the platelet extraction syringe coupled to the support portion 35.

In addition, the communication hole 36 has a much smaller volume than the interior of the housing 10, whereby, when blood is moved into the plasma extraction syringe or the platelet extraction syringe coupled to the support portion 35, the boundary between plasma and platelets or between platelets and red blood cells can be easily identified.

The connector 40 includes a generally cylindrical connection body 41, a male thread portion 42 protruding from a lower end of the connection body 41 and having a threaded outer surface to be detachably coupled to an upper portion of the support portion 35, and a female thread portion 43 recessed from an upper surface of the connection body 41 and having a threaded inner surface to allow the plunger 50 described below to be detachably coupled thereto. The connector 40 serves to connect the collector 30 press-fitted into the housing 10 to the plunger 50, thereby allowing a blood collection operation to be performed.

In addition, the connector 40 is separated from the collector 30 after completion of a blood centrifugation operation. Preferably, the connector 40 has such a length that an upper portion thereof partially protrudes outside the housing 10 after blood collection so as to facilitate separation of the connector 40.

The plunger 50 has a shape corresponding to the shape of a plunger 50 of a typical syringe and has a fastening portion 51 formed at a tip thereof to be coupled to the female thread portion 43.

The plunger 50 is coupled to the collector 30 upon trying to collect blood, thereby allowing a blood collection operation to be performed.

The holder 60 includes a holder body 61 having an inner space 61a, open at a top thereof, and having a side surface formed with an insertion hole 61b through which the housing 10 is inserted into the holder body 61 and a support cover 62 coupled to an upper portion of the holder body 61, having an insertion groove 62a formed at one side thereof corresponding to the insertion hole 61b, and supporting one side of the housing 10 on an upper surface thereof.

That is, the holder 60 is configured such that the housing 10 is inserted thereinto through the insertion hole 61b and the insertion groove 62a and one side of the upper portion of the inserted housing 10 is supported on the upper surface of the support cover 62. When the plasma extraction syringe or the platelet extraction syringe is coupled to the housing 10 and a main body 21 of the corresponding syringe is pressed downward to extract plasma or platelets from blood in the housing 10, the holder 60 allows the housing 10 to be supported on the ground, thereby allowing stable plasma or platelet extraction operation.

In addition, the holder 60 further includes a movement prevention portion formed at one side of an inner surface thereof to support one side of the outer surface of the housing 10 retained in the holder 60, thereby preventing movement of the housing 10 during PRP or PPP extraction.

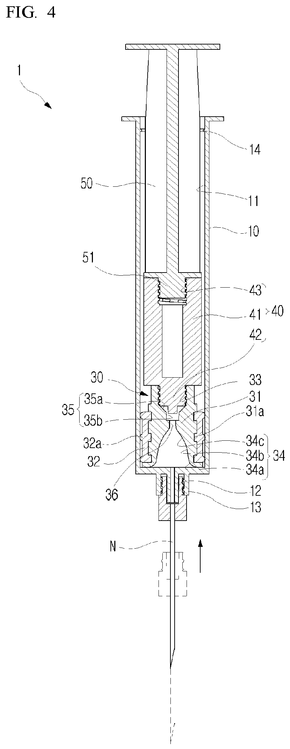

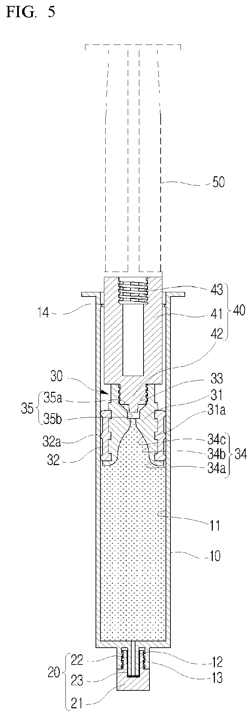

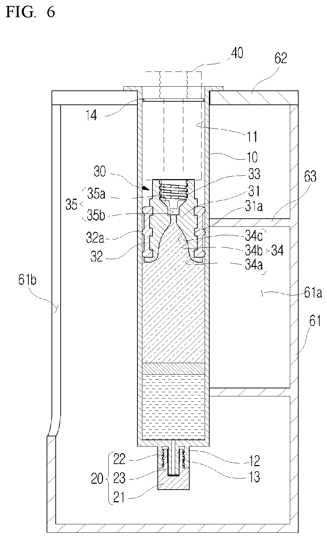

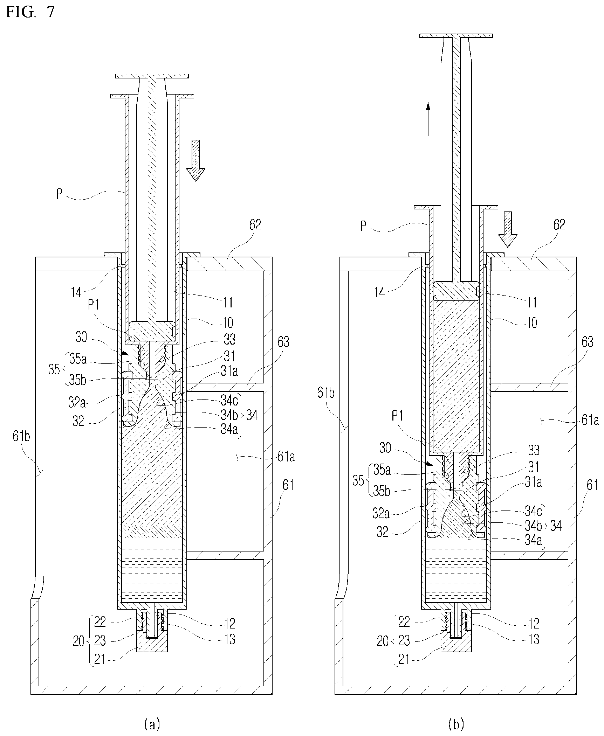

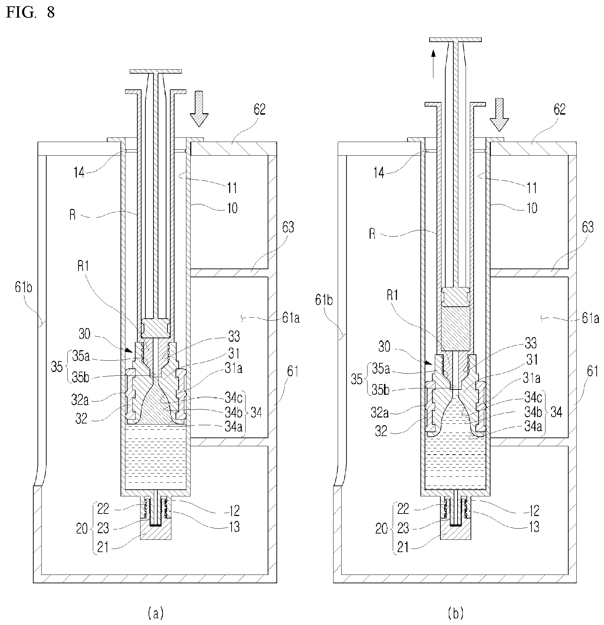

FIG. 4 is a sectional view of the syringe-integrated platelet extraction device of FIG. 1, with a syringe needle coupled to the housing thereof, FIG. 5 is a sectional view of the syringe-integrated platelet extraction device of FIG. 1, with blood collected in the housing thereof, FIG. 6 is a sectional view of the syringe-integrated platelet extraction device of FIG. 1, showing centrifugation of blood collected in the housing thereof, FIG. 7 is a sectional view of the syringe-integrated platelet extraction device of FIG. 1, showing extraction of plasma in the housing thereof, and FIG. 8 is a sectional view of the syringe-integrated platelet extraction device of FIG. 1, showing extraction of platelets in the housing thereof.

Now, a method of extracting platelets using the syringe-integrated platelet extraction device 1 according to the embodiment of the present invention will be described with reference to FIG. 4 to FIG. 8.

First, the syringe-integrated platelet extraction device 1 according to the embodiment, a plasma extraction syringe P, and a platelet extraction syringe R are prepared.

A needle N is coupled to a leading end of the housing 10 and the plunger 50 is coupled to the connector 40 at a trailing end of the housing 10, followed by collecting blood from a subject. Here, 20 cc of blood is generally collected to extract 1 cc of platelets. In this embodiment, upon completion of blood collection, the upper portion of the connector 40 protrudes outside the housing 10 (see FIG. 5).

After completion of blood collection, the needle N and the plunger 50 are separated from the housing 10 and then the cap 20 is screwed onto the leading end of the housing 10 to seal the housing 10.

The sealed housing 10 is mounted on a centrifuge, followed by centrifugation to separate the blood into plasma, platelets, and red blood cells.

Thereafter, the housing 10 is inserted into the holder 60 through the insertion hole 61b and the insertion groove 62a of the holder 60 and then one side of the upper portion of the housing 10 is held on the upper surface of the support cover 62, as shown in FIG. 6. With the upper portion of the housing 10 held on the upper surface of the support cover 62, one side of the outer surface of the housing 10 is supported against the movement prevention portion 63, whereby the housing 10 can be more stably retained in the holder 60.

Then, after separating the connector 40 from the collector 30 (see FIG. 6), the tip of the plasma extraction syringe P is inserted into and coupled to the support portion 35 (see FIG. 7(a)). Even when an orifice PI of the plasma extraction syringe P is not accurately inserted into the second coupling portion 35b, the inclined surface of the first coupling portion 35a can guide the orifice P1 toward the second coupling portion 35b, thereby allowing the orifice PI to be easily inserted into and coupled to the second coupling portion 35b.

With the plasma extraction syringe P inserted into and coupled to the support portion 35, an outer periphery of the plasma extraction syringe P is brought into surface contact with and supported by the first coupling portion 35a and the orifice PI is inserted into and coupled to the second coupling portion 35b, whereby the plasma extraction syringe P can be firmly connected to and held against the collector 30.

After connecting the plasma extraction syringe P to the collector 30, a main body 21 of the plasma extraction syringe P is pressed downward to extract the plasma contained in the housing 10.

Since the communication hole 36 located on a movement path of the plasma has a smaller diameter than the housing 10 and has a predetermined height in a vertical direction, the boundary between plasma and platelets can be easily identified through the communication hole 36, thereby allowing precise extraction of the plasma.

In addition, according to this embodiment, since the cup portion 34 is designed to have a volume corresponding to the amount of platelets to be extracted, a PRP layer can be precisely charged in the cup portion 34 by ceasing a plasma extraction operation at the time that the reception groove 34 is filled up with platelets during plasma extraction.

In the vicinity of the boundary between plasma and platelets, partial mixing between the plasma and the platelets occurs, causing reduction in platelet purity. Accordingly, it is desirable that an additional 1 cc to 2 cc of platelets be extracted along with plasma during plasma extraction.

After completion of plasma extraction, the plasma extraction syringe P is separated from the collector 30 and the platelet extraction syringe R is inserted into and coupled to the collector 30 in the same manner as the plasma extraction syringe P (see FIG. 8).

Here, the PRP layer can be naturally moved from the cup portion 34 toward the support portion 35, that is, toward the platelet extraction syringe R along the first curved portion 34a, the inclined portion 34b, and the second curved portion 34c, thereby minimizing occurrence of a vortex during movement of platelets and thus allowing extraction of high-purity platelets free from admixed red blood cells.

Although some exemplary embodiments have been described herein, it should be understood by those skilled in the art that these embodiments are given by way of illustration only, and that various modifications, variations and alterations can be made without departing from the spirit and scope of the invention. Therefore, the scope of the invention should be interpreted according to the following appended claims as covering all modifications or variations derived from the appended claims and equivalents thereto.

## Claims

1. A syringe-integrated platelet extraction device comprising:
a housing having a longitudinal inner space for receiving collected blood, open at a top thereof, and formed at a bottom thereof with an orifice to which a syringe needle is coupled;
a cap coupled to the orifice of the housing;
a collector press-fitted into the housing from above the housing;
a connector detachably coupled to an upper portion of the collector; and
a plunger detachably coupled to the connector,
wherein the collector comprises a body movably disposed inside the housing, a coupling portion formed at an upper side of the body and allowing a lower end of the connector to be detachably coupled thereto, a cup portion inwardly recessed from a lower surface of the body and receiving platelets therein, a support portion extending from a lower end of the coupling portion and allowing a plasma extraction syringe or a platelet extraction syringe to be inserted thereinto and coupled thereto, and a communication portion having a smaller diameter than the support portion and allowing the cup portion to communicate with the support portion therethrough.

2. The syringe-integrated platelet extraction device according to claim 1, wherein the cup portion comprises:
a first curved portion formed at a lower inner periphery of the body to be curved in an inward direction of the body;
an inclined portion upwardly extending from the first curved portion and inclined at a predetermined angle toward the support portion; and
and a second curved portion extending from an upper end of the inclined portion and curved toward the support portion.

3. The syringe-integrated platelet extraction device according to claim 1 or 2, wherein the cup portion has a volume corresponding to an amount of platelets to be extracted.

4. The syringe-integrated platelet extraction device according to any one of claims 1 to 3, wherein the support portion comprises:
a first coupling portion downwardly extending from the coupling portion, tapered toward a lower end thereof, and supporting an outer periphery of a tip of the plasma extraction syringe or the platelet extraction syringe; and
a second coupling portion downwardly extending from a lower end of the first coupling portion, having a smaller diameter than the first coupling portion, and allowing an orifice of the plasma extraction syringe or the platelet extraction syringe to be inserted thereinto and coupled thereto.

5. The syringe-integrated platelet extraction device according to any one of claims 1 to 4, wherein the collector further comprises a soft gasket coupled to an outer surface of the body.

6. The syringe-integrated platelet extraction device according to claim 5, wherein the gasket has multiple annular protrusions formed on an outer surface thereof.

7. The syringe-integrated platelet extraction device according to any one of claims 1 to 6, wherein the housing further comprises an annular ledge inwardly protruding from an upper inner surface thereof.

8. The syringe-integrated platelet extraction device according to any one of claims 1 to 7, further comprising:
a holder retaining the housing by allowing one side of an outer surface of the housing to be supported on one side of an upper surface thereof.

9. The syringe-integrated platelet extraction device according to claim 8, wherein the holder comprises a movement prevention portion formed at one side of an inner surface thereof to support the one side of the outer surface of the housing.
